# EUROPÄISCHE PATENTANMELDUNG

(11) **EP 4 497 656 A1**
(43) Veröffentlichungstag der Anmeldung: **29.01.2025**
(21) Anmeldenummer: 24190434.1
(22) Anmeldetag: 23.07.2024
(51) Int. Cl.: B62B 3/14, A61L 9/04, A61L 9/12, G09F 19/00, A61L 9/013

(54) **EINKAUFSBEHÄLTER**

(30) Priorität: 24.07.2023 DE 202023104118 U
(71) Anmelder: Kador, Utz, 6020 Innsbruck (AT)
(72) Erfinder: Kador, Utz, 6020 Innsbruck (AT)
(74) Vertreter: Kador & Partner Part mbB

(57) **Zusammenfassung**

Einkaufsbehälter (1) dadurch gekennzeichnet, dass dieser einen Riechstoff abgibt oder dieser eine mit dem Einkaufsbehälter(1) verbundene Einrichtung (2) aufweist, die einen Riechstoff abgibt.

## Beschreibung

Die Erfindung betrifft einen Einkaufsbehälter. Unter Einkaufsbehälter werden in erster Linie Einkaufswägen sowie ziehbare und tragbare Einkaufskörbe verstanden.

Es ist bekannt Geschäftsräume, wie Supermärkte mit Düften zu versehen, um den Kunden ein angenehmes Gefühl zu vermitteln. Nachteilig dabei ist, dass Supermärkte sehr groß sind und hohe Raumhöhen haben, sodass für dieses sehr große Volumen an Raumluft eine große Menge an teurem Riechstoff benötigt wird, um einen Effekt zu erzielen. Leicht flüchtige Riechstoffe konzentrieren sich im Deckenbereich wo sie keine Wirkung entfalten.

Aufgabe der Erfindung ist es, eine Verbesserung der Wahrnehmung von Gerüchen bei deutlich kleineren Mengen an benötigtem Riechstoff zu erreichen.

Der Erfindung liegt die Erkenntnis zugrunde, dass der Riechstoff in die Nähe des Kunden gebracht wird. Dazu eignet sich der Einkaufsbehälter, wie ein Einkaufswagen, weil dieser sich meist in der Nähe des Kunden befinden.

Gegenstand der Erfindung ist ein Einkaufsbehälter der einen Riechstoff abgibt oder der eine Einrichtung aufweist, die mit dem Einkaufsbehälter verbunden ist und einen Riechstoff abgibt.

Zahlreiche Riechstoffe entfalten für den Menschen angenehme Gefühle und das ist erwünscht, denn der Kunde soll sich im Geschäft, im Supermarkt wohl fühlen. Auf diese Weise kann eine gewisse Kundenbindung oder auch Geschäftsidentität erreicht werden.

Ein angenehmer Geruch bewirkt beim Menschen eine positive Reaktion des Gehirns, ohne dass es dem Menschen auffällt, weil der Geruchssinn über das Gehirn unbewusst wirkt.

Geeignete Riechstoffe können aber auch unangenehme Gerüche überlagern, die zB. von Verpackungen, Reinigungsmitteln oder Konservierungs- oder anderen Schutzmitteln stammen, und so eine angenehmere Atmosphäre schaffen.

Geeignete Riechstoffe können natürliche, künstliche oder synthetische und auch naturidentische Riechstoffe sein. Sie müssen sich in Luft verteilen also flüchtig sein.

Die Riechstoffe müssen gerochen werden, also in ausreichender Konzentration im Bereich der Nase des Kunden vorliegen, jedenfalls muss die Konzentration oberhalb des Geruchsschwellerwertes liegen.

Geeignete Riechstoffe sind beispielsweise Rosendüfte, Geranien- oder Muskatellersalbei Düfte. Zitrusfrüchte Düfte wirken erfrischend und der Duft der Vanille erzeugt Wohlbefinden

Wenn erfindungsgemäss der Riechstoff vom Einkaufsbehälter abgegeben wird sind viel geringere Mengen an Riechstoff notwendig, weil es ausreicht wenn nur im Bereich des Einkaufsbehälters genug Riechstoff vorliegt, bzw. die Konzentration an Riechstoff ausreichend hoch ist, da sich der Kunde meist beim Einkaufsbehälter aufhält, den Einkaufswagen schiebt oder den Einkaufskorb trägt.

Im Bereich des Einkaufsbehälters, insbesondere in einem Bereich von 0,5 bis 2m um den Einkaufsbehälter soll die Konzentration des Riechstoffes zumindest oberhalb des Geruchschwellenwertes liegen.

Wenn nicht, wie gemäß dem Stand der Technik die gesamte Raumluft mit Riechstoff belegt ist, sondern gemäß der Erfindung vorgegangen wird, kann der Kunde wenn er sich vom Einkaufswagen weg zur Brottheke oder Käsetheke bewegt den Duft von Brot oder Käse wahrnehmen.

Insgesamt wird erreicht, dass der Kunde sich im Supermarkt wohl fühlt, tendenziell im Supermarkt länger verweilt und dank der positiven Geruchserinnerung bestrebt ist, in den gleichen Supermarkt zurückzukehren.

Der Riechstoff kann direkt in den Einkaufsbehälter eingebracht werden. Beispielsweise kann der Riechstoff in die Schiebestange eines Einkaufswagens eingebracht werden. Dazu kann die Schiebestange mit einer Öffnung, die vorzugsweise verschließbar ist, versehen werden und der Riechstoff wird in einem geeigneten Trägermaterial, wie Watte, eingebracht. Die Schiebestange weißt kleine Öffnungen auf durch die der Riechstoff entweicht.

Vorzugsweise wird der Riechstoff aber in eine Riechstoff abgebende Einrichtung eingebracht, die mit dem Einkaufsbehälter verbunden wird. Diese Einrichtung weist eine Öffnung zum Einbringen des Riechstoffs, vorzugsweise auf einem Träger, und vorzugsweise kleine Öffnungen zum Entweichen des Riechstoffs auf, die vorzugsweise verschließbar sind, um das Entweichen in Ruhezeiten, wie Nacht oder Wochenende, zu vermeiden.

Die Erfindung wird nachstehend anhand der Zeichnung näher erläutert.

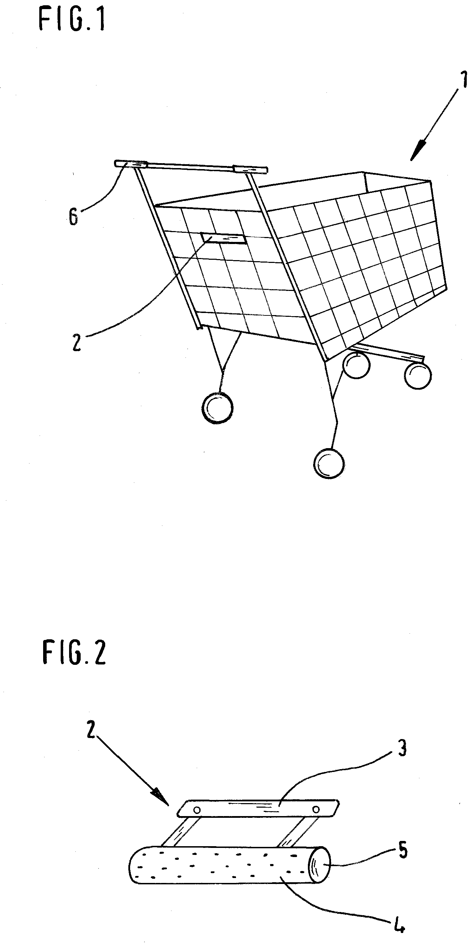

Die Figur 1 zeigt einen Einkaufsbehälter (1), nämlich einen Einkaufswagen, der an der Vorderseite unterhalb Schiebestange (6) eine Riechstoff abgebende Einrichtung (2) trägt.

Diese Einrichtung (2) ist in Figur 2 gezeigt. Sie weist eine Öffnung (5) zum Einbringen der Riechstoffs und kleine Öffnungen (4) zum Entweichen des Riechstoffs auf.

Die Einrichtung (2) weist ferner eine Halterung (3) auf mit der die Einrichtung (2) am Einkaufsbehälter (1) befestigbar ist, aber auch leicht wieder gelöst werden kann.

Die kleinen Öffnungen (4) können beispielsweise mit einer Hülle aus Kunstoff ( nicht gezeigt) verschlossen werden.

### Bezugszeichenliste

1 Einkaufsbehälter
2 Riechstoffe abgebende Einrichtung
3 Halterung
4 kleine Öffnungen
5 Öffnung der Einrichtung
6 Schiebestange

## Patentansprüche

1. Einkaufsbehälter (1) **dadurch gekennzeichnet, dass** dieser einen Riechstoff abgibt oder dieser eine mit dem Einkaufsbehälter(1) verbundene Einrichtung (2) aufweist, die einen Riechstoff abgibt.

2. Einkaufsbehälter nach Anspruch 1, **dadurch gekennzeichnet, dass** die Riechstoff abgebende Einrichtung (2) lösbar am Einkaufsbehälter (1) befestigbar ist.

3. Einkaufsbehälter nach Anspruch 1 oder 2, **dadurch gekennzeichnet dass** die den Riechstoff abgebende Einrichtung (2) eine Halterung (3) aufweist, mit der sie am Einkaufsbehälter (3) befestigbar ist.

4. Einkaufsbehälter nach einem der Ansprüche 1 bis 3, **dadurch gekennzeichnet dass** die Riechstoff abgebende Einrichtung Öffnungen (4) aufweist.

5. Einkaufsbehälter nach Anspruch 4, **dadurch gekennzeichnet dass** die Öffnungen (4) verschließbar sind.

6. Einkaufsbehälter nach einem der Ansprüche 1 bis 5, **dadurch gekennzeichnet dass** der Riechstoff ein Rosen-, Geranien-, Muskatellersalbei- und/oder Vanille-Riechstoff ist.

7. Einkaufsbehälter nach einem der Ansprüche 1 bis 6, **dadurch gekennzeichnet dass** die Konzentration des Riechstoffs in einem Bereich von 0,5 bis 2m um den Einkaufsbehälter (1) oberhalb des Geruchsschwellenwertes liegt.
